# EUROPEAN PATENT APPLICATION

(11) **EP 4 714 457 A1**
(43) Date of publication of application: **25.03.2026**
(21) Application number: 23937649.4
(22) Date of filing: 29.12.2023
(51) Int. Cl.: A61K 35/00, A61K 35/12, A61K 35/14, A61K 35/66, A61K 35/74, A61K 35/76, A61K 38/17, C12N 5/07

(54) **METHOD FOR PRODUCING A CONDITIONED BLOOD COMPOSITION FOR TREATING DISEASES**

(30) Priority: 15.05.2023 KZ 20230518
(71) Applicant: Yucht, Roman, AImaty, 050010 (KZ)
(72) Inventor: Yucht, Roman, AImaty, 050010 (KZ)
(74) Representative: Lapienis, Juozas
(86) International application number: PCT/KZ2023/000026
(87) International publication number: WO 2024/237773

(57) **Abstract**

The invention relates to medicine and more particularly to blood-based medicinal preparations containing induced cytokines for treating human diseases. In a method for producing a conditioned blood composition for treating diseases, including collecting blood from a patient, incubating the collected blood in a vessel, centrifuging, according to the invention, the collected blood of the patient is mixed with a vaccine containing viral fragments, and/or with a vaccine containing microbial toxins, and/or with a vaccine containing attenuated microbial DNA, followed by incubation, centrifugation to obtain a cytokine-enriched conditioned blood composition. In the method for producing a conditioned blood composition the collected blood of the patient is mixed with the Vaxigrip influenza vaccine or with the Influvac Tetra vaccine, and/or with a hepatitis B vaccine, and/or with a tetanus vaccine, and/or with a rubella vaccine. The developed method for producing a conditioned blood composition for treating diseases provides an increased activation rate, as well as a high content of interleukin B in the conditioned blood composition.

## Description

The invention relates to medicine and more particularly to blood-based medicinal preparations containing induced cytokines for treating human diseases.

A method is known for producing a conditioned blood composition containing a therapeutically and prophylactically highly effective mixture of biologically active substances, such as cytokines and factors, for example, TNFa, interleukins IL-1Ra, IL-6, IL-4, IL-10 IL-13, intended for treating muscular, musculoskeletal diseases, inflammations and irritations of the nervous system, autoimmune diseases, in particular rheumatoid arthritis, neurodermatitis-caused diseases, as well as for treating and healing chronic wounds, in particular diabetic ulcers, for treating endometriosis (EAPO pat. 014435, cl. A61K35/14, A61P37/00, publ. 30.12.2010, patent holder Ortogen AG, DE), prototype.

The method is carried out as follows. The patient's blood is collected and incubated in a modified vessel at a temperature of 10-40° for 2-36 hours under an oxygen partial pressure of less than 5 kPa. The modified vessel has an internal structure with a large surface area created by the use of, for example, balls, fibers, flour; glass beads can be placed inside the vessel. In an additional step, the cellular constituents are separated from the liquid constituents by centrifugation, and a conditioned blood serum composition is obtained. The composition obtained by this method contains 30-20,000 pg/ml of interleukin 6, IL-6, and additional components, for example IL-10, IL-13, insulin-like growth factor PDGF.

In this method for producing a conditioned blood composition, it has been proposed to use mechanical action on the blood in the form of providing a non-smooth inner surface of the vessel for processing, as well as placing glass beads in the vessel, which does not promote sufficiently effective blood activation, both in terms of cytokine production time and interleukin 6 concentration.

The objective of the proposed invention and the technical result is the development of a method for producing a conditioned blood composition for treating diseases, which ensures an increased activation rate, as well as a high content of interleukin 6 in the conditioned blood composition.

In order to achieve the technical result in the method for producing a conditioned blood composition for treating diseases, including collecting blood from a patient, incubating the collected blood in a vessel, centrifuging, according to the invention, the collected blood of the patient is mixed with a vaccine containing viral fragments, and/or with a vaccine containing microbial toxins, and/or with a vaccine containing attenuated microbial DNA, followed by incubation, centrifugation to obtain a cytokine-enriched conditioned blood composition.

In the method for producing a conditioned blood composition, the collected blood of the patient is mixed with the Vaxigrip influenza vaccine or with the Influvac Tetra vaccine, and/or with a hepatitis B vaccine, and/or with a diphtheria and tetanus vaccine, and/or with a measles, mumps, and rubella vaccine.

In the method for producing a conditioned blood composition, incubation is carried out at a temperature of 25-40°, preferably at 38°.

In the method for producing a conditioned blood composition, the obtained cytokine-enriched blood composition is mixed with a leukocyte- and platelet-enriched blood fraction obtained by centrifugation of blood collected from the patient 6-24 h after the initial blood collection.

In the method for producing a conditioned blood composition, the cytokine-enriched fraction is mixed with steroid hormones.

In the Vaxigrip vaccine (0.5 ml) used, one dose contains inactivated split influenza viruses represented by strains equivalent to the following:
A(H₁N₁)** 15 µg GA
A(H₃ N₂ )** 15 µg GA
B** 15 µg GA
* the field contains the names of the strains recommended by the WHO for the current influenza epidemic season.
** hemagglutinin.

One dose of the Influvac Tetra vaccine (0.5 ml) contains hemagglutinin and neuraminidase of the following viral strains:
A(H₃N₂)** 15 µg GA
A(H₁ N₁)** 15 µg GA
B** 15 µg GA
* were cultured on chicken embryos of healthy chickens, as well as excipients.

The available hepatitis B vaccines are single-antigen vaccines, Engerix-B and Recombivax HB, containing aluminium adjuvants. The latest HepB-CpG formula uses thecytidine-phosphate-guanosine oligodeoxynucleotide (CpG-ODN) immunostimulatory adjuvant. A combination vaccine that combines hepatitis A and hepatitis B vaccines (Twinrix) is also available.

The measles, mumps, and rubella vaccine contains attenuated strains of measles, rubella, and mumps viruses with excipients: an aqueous solution LS-18, a gelatin solution and gentamicin sulfate.

The diphtheria and tetanus vaccine, DTP, is an adsorbed combination vaccine that contains killed whole pertussis bacterium (therefore it is also called a whole-cell vaccine), along with diphtheria toxoid (inactivated toxin) and tetanus toxoid.

In the proposed method for producing a conditioned blood composition, blood is activated using microbial and viral metabolic products, and the use of ready-made vaccine formulations for activation is a significant feature of the technology, which increases the activation rate and ensures a high content of interleukin 6 in the conditioned blood composition.

Compared to mechanical processing of blood according to the prototype, biological activation of blood made it possible to obtain a conditioned blood composition with high cytokine concentration characteristics and to increase the activation rate.

Mixing the resulting cytokine-enriched blood composition with a leukocyte- and platelet-enriched blood fraction is carried out in the case of, for example, treating narrowing of the spinal canal or foraminal canal.

Mixing the resulting cytokine-enriched blood composition with corticosteroid hormones is carried out in the case of, for example, diseases associated with problems of tendons or lateral ligaments.

We compared interleukin 1-1b, interleukin 6 and interleukin 10 in the conditioned blood composition obtained by the proposed method with those in patient-derived blood incubated at 37-38°. It was determined that in the proposed blood composition, interleukin 1-1b is 100 times higher than its normal plasma concentration and 10 times higher than concentration in blood incubated at 37-38°. Interleukin 6 is 90 times higher than freshly punctured blood and five times higher than heat-treated blood, interleukin 10 is 10 times higher than normal blood and 7 times higher than heat-treated blood. These data are very important because they make it possible to observe the difference between normal blood and conditioned blood composition with the addition of vaccines.

The proposed method for producing a conditioned blood composition is carried out as follows.

Using a butterfly needle, a venipuncture is performed and the patient's venous blood is collected into a 10 ml test tube. Then 0.2 ml of the Vaxigrip influenza vaccine or the Influvac Tetra vaccine is added with an insulin needle into a test tube containing the collected blood. The test tube is shaken and placed in a thermostat for 6-24 hours (depending on the amount of desired cytokines) at a temperature of 38°. Then centrifugation is carried out at 3,500 rpm to obtain the plasma separated from the dense blood particles. Cytokine-enriched plasma is ready for use in treatment. In the same manner, the measles, mumps, and rubella vaccine, the hepatitis B vaccine, the diphtheria and tetanus vaccine, and mixed vaccines were added to venous blood.

6-24 hours after the initial blood collection, the patient undergoes venipuncture and collection of venous blood in an amount of 5-60 ml. Centrifugation is carried out in one, two, or three stages, and the blood fraction containing concentrated immune system cells - leukocytes, platelets - is separated and mixed with the obtained cytokine-enriched composition.

The conditioned blood composition is mixed with corticosteroid hormones, such as triamcinolone.

The conditioned blood composition produced by the proposed method was tested for treating human diseases, but can be used for treating animals as well as in cosmetology.

Example 1. A 41-year-old man born in 1981, diagnosis: lateral epicondylitis of the left elbow, complaints: has been suffering for more than 6 months from pain in the left elbow during dumbbell workouts, as well as from pain in the left elbow when shaking hands.

Treatment: one injection of the proposed cytokine-enriched blood composition into the external condyle of the left humerus. Dynamics: after one month, the pain completely disappeared, control: after 6 months, the pain did not return.

Example 2. A man born in 1986, complaints: has been suffering from pain in the right knee for more than three years, when going up and down the stairs. Diagnosis: complete rupture of the posterior horn of the medial meniscus of the right knee joint.

Treatment: injection of the proposed blood composition mixed with a leukocyte- and platelet-enriched blood fraction, using ultrasound guidance into the posterior horn of the meniscus once a month, for a total of two times. Dynamics: MRI shows the formation of fibrocartilage at the site of the meniscus tear. The patient reported complete disappearance of pain when going up the stairs.

Example 3. A 59-year-old man born in 1963, diagnosis: plantar fasciitis (heel spur). Complaints: severe pain in both heels, more in the left than in the right, VAS 9/10 when stepping in the mornings, first steps.

Treatment: injection of the proposed cytokine-enriched blood composition supplemented with 20 milligrams of triamcinolone.

Control. After 3 days, the pain completely disappeared, after 6 months, there was no recurrence of pain.

Example 4. A 72-year-old woman born in 1950. Complaints: lower back pain radiating to the legs for more than 5 years, noticeable worsening over the past year, unable to walk independently for more than 10, 20 meters due to radiating pain and weakness in the legs. Diagnosis: stenosis of L4, L5 due to degenerative changes in the spine by about 90-95 percent of the volume.

Treatment: three injections of the proposed conditioned blood composition, epidurally into the L4, L5 region under MRI guidance. Each injection was given at intervals of 3-4 weeks.

Result. One week after the start of treatment, she was able to walk more than 100 meters; after 6 months, she walks more than a kilometer.

The proposed method for producing a conditioned blood composition for treating diseases provides the following advantages.
1. The price is low relative to the market; for example, compared to the blood composition based on the Orthokine prototype, the price is about 20 times lower, that is, 20 times cheaper.
2. The possibility of creating a cytokine-enriched blood composition corresponding to different treatment purposes and to the activation of immune system response processes in the human or animal body, for treatment, recovery, regeneration or cosmetic purposes.
3. We compared interleukin 1-1b, interleukin 6 and interleukin 10 in the conditioned blood composition obtained by the proposed method with those in blood collected from a patient and incubated at 38°. It was determined that in the proposed blood composition, interleukin 1-1b is 100 times higher than its normal plasma concentration and 10 times higher than concentration in blood incubated at 38°. Interleukin 6 is 90 times higher than its content in normal blood and five times higher than in heated blood, interleukin 10 is 10 times higher than its content in normal blood and 7 times higher than in heated blood.
4. Ease of use, simple and inexpensive equipment such as centrifuges and syringes.
5. High cytokine concentration in plasma, as mentioned in section three.

## Claims

1. A method for producing a conditioned blood composition for treating diseases, including collecting blood from a patient, incubating the collected blood in a vessel, centrifuging, **characterized in that** the collected blood of the patient is mixed with a vaccine containing viral fragments, and/or with a vaccine containing microbial toxins, and/or with a vaccine containing attenuated microbial DNA, followed by incubation, centrifugation to obtain a cytokine-enriched conditioned blood composition.

2. The method for producing a conditioned blood composition according to claim 1, **characterized in that** the collected blood of the patient is mixed with the Vaxigrip influenza vaccine or with the Influvac Tetra vaccine, and/or with a hepatitis B vaccine, and/or with a diphtheria and tetanus vaccine, and/or with a measles, mumps, and rubella vaccine.

3. The method for producing a conditioned blood composition according to claim 1, **characterized in that** the incubation is carried out at a temperature of 25-40°, preferably at 38°.

4. The method for producing a conditioned blood composition according to claim 1, **characterized in that** the obtained cytokine-enriched conditioned blood composition is additionally mixed with the leukocyte- and platelet-enriched blood fraction obtained by centrifugation of blood collected from the patient 6-24 hours after the initial blood collection.

5. The method for producing a conditioned blood composition according to claim 1, **characterized in that** the cytokine-enriched conditioned blood composition is additionally mixed with steroid hormones.
